# EUROPÄISCHE PATENTANMELDUNG

(11) **EP 1 669 027 A1**
(43) Veröffentlichungstag der Anmeldung: **14.06.2006**
(21) Anmeldenummer: 06006663.6
(22) Anmeldetag: 24.01.1998
(51) Int. Cl.: A61B 5/107, A61F 2/30

(54) **Verfahren und Vorrichtung zur präoperativen Bestimmung der Positionsdaten von Endoprothesenteilen**

(30) Priorität: 11.03.1997 DE 19709960
(62) Teilanmeldung aus: 02013129.8
(71) Anmelder: Aesculap AG & Co. KG, 78532 Tuttlingen (DE)
(72) Erfinder: Cinquin, Philippe, 38700 La Tronche (FR); Lavalle, Stéphane, 38660 Saint Vincent-de Mercuze (FR); Leitner, Francois, 38410 Uriage (FR); Minfelde, Richard, 75019 Paris (FR); Picard, Frédéric, 77630 Barbizon (FR); Saragaglia, Dominique, 38640 Claix (FR); Schulz, Hans-Joachim, 78532 Tuttlingen (DE)
(74) Vertreter: Hoeger, Stellrecht & Partner Patentanwälte

(57) **Zusammenfassung**

Um bei einem Verfahren zur präoperativen Bestimmung der Positionsdaten von Endoprothesenteilen eines mittleren Gelenkes (4) relativ zu einem der beiden das mittlere Gelenk ausbildenden Knochen (5,6) die Lage des Knochens im Körper ohne komplizierte Verfahren feststellen zu können, wird vorgeschlagen, dass man die an einem äußeren Gelenk, das sich an dem dem mittleren Gelenk abgewandten Ende der beiden das mittlere Gelenk bildenden Knochen befindet, zusammenkommenden Knochen (5,19;6,20) gegeneinander bewegt und dadurch für einen der beiden das mittlere Gelenk bildenden Knochen einen Gelenkpunkt bestimmt, dass man im Bereich des mittleren Gelenkes für einen der beiden das mittlere Gelenk bildenden Knochen einen Gelenkpunkt bestimmt, dass man aus den Positionsdaten des so gewonnenen äußeren Gelenkpunktes und des so gewonnenen Gelenkpunktes im Bereich des mittleren Gelenkes die Orientierung der Endoprothesenteile relativ zu einem der beiden das mittlere Gelenk bildenden Knochen bestimmt.

## Beschreibung

Die Erfindung betrifft ein Verfahren zur präoperativen Bestimmung der Positionsdaten von Endoprothesenteilen eines mittleren Gelenkes relativ zu den das mittlere Gelenk ausbildenden Knochen. Außerdem betrifft die Erfindung eine Vorrichtung zur Durchführung dieses Verfahrens.

Bei chirurgischen Operationen, bei denen Gelenke zwischen zwei Knochen durch Endoprothesen ersetzt werden müssen, ist es äußerst wichtig, daß die Endoprothesenteile relativ zu den Knochen exakt positioniert werden, Abweichungen in der Größenordnung von mehr als 2° stellen den Erfolg einer solchen Operation bereits infrage.

Es ist bekannt, zur Vorbereitung von chirurgischen Operationen die Lage von Knochen im Körper und die relative Positionierung der an das zu ersetzende Gelenk angrenzenden Knochen durch verschiedene Verfahren zu bestimmen, um vor der Operation bereits planen zu können, wie die Endoprothesenteile relativ zu den Knochen eingesetzt werden müssen. Beispielsweise ist es bekannt, die Außenkontur der an das zu ersetzende Gelenk angrenzenden Knochen durch Computertomographieaufnahmen zu bestimmen, anhand der so gewonnenen Daten lassen sich Datensätze erstellen, die den Außenkonturen der Knochen entsprechen und die dann zur Planung der Orientierung der Prothesenteile benutzt werden können (M. Fadda et al "Computer-Assisted Knee Arthoplasty at Rizzoli Institute"; MRCAS 94, Medical Robotics and Computer Assisted Surgery, Pittsburgh, 1994, Seiten 26 bis 31; T. C. Kienzle III et al "A Computer-Assisted Total Knee Replacement Surgical System Using a Calibrated Robot", MIT Press, Cambridge, MA, 1996, Seiten 409 bis 416).

Dies setzt eine komplizierte Untersuchung des Patienten vor Beginn der Operation voraus, die häufig nicht am eigentlichen Operationsort und daher in der Regel auch nicht zeitgleich mit der Operation durchgeführt werden kann. Außerdem muß der Patient dabei einer hohen Strahlendosis ausgesetzt werden, schließlich sind für diese Untersuchung teure apparative Ausstattungen notwendig.

Es ist bereits bekannt, die Lage der Knochen vor und nach der Operation dadurch miteinander zu vergleichen, daß an den Knochen Markierungselemente befestigt werden, deren Position im Raum durch geeignete kamera-ähnliche Vorrichtungen bestimmt werden kann (US-A-5,249,581). Mit einer solchen Vorrichtung kann das Ergebnis der Operation geprüft werden, denn der Operateur kann die Orientierung der Knochen vor und nach der Operation vergleichen. Es ist jedoch mit diesem Verfahren nicht möglich, präoperativ die Positionsdaten der einzusetzenden Prothesenteile zu bestimmen, auch bei diesem Verfahren muß die Lage der Prothesenteile am Knochen präoperativ dadurch bestimmt werden, daß zum Beispiel durch Computertomographie-Aufnahmen die genaue Lage der Knochen im Körper und ihre Relativpositionierung zueinander bestimmt werden.

Es ist Aufgabe der Erfindung, ein Verfahren anzugeben, mit dem präoperativ die Lage der Prothesenteile relativ zu dem Knochen bestimmt werden kann, ohne daß dazu komplizierte Untersuchungsverfahren des Patienten notwendig werden, insbesondere sollen CT-Aufnahmen oder ähnliche Untersuchungsverfahren überflüssig werden.

Diese Aufgabe wird durch das Verfahren gemäß Anspruch 1 gelöst.

Das beschriebene Verfahren läßt sich an allen Körperteilen anwenden, bei denen die das zu ersetzende Gelenk bestimmenden Knochen an ihrem anderen Ende ebenfalls über ein Gelenk mit weiteren Knochen verbunden sind. Nachfolgend wird das zu ersetzende Gelenk als "mittleres Gelenk" bezeichnet, die außenseitig anschließenden Gelenke als "äußere Gelenke". Bei dem beschriebenen Verfahren werden nun die äußeren Gelenke dazu verwendet, präoperativ Informationen über die Lage der am mittleren Gelenk anschließenden Knochen zu liefern. Es werden nämlich die an den beiden äußeren Gelenken zusammenkommenden Knochen gegeneinander bewegt, und durch diese Bewegung wird die Lage der äußeren Gelenke bestimmt, genauer Gelenkpunkte größer Invarianz. Dies wird am Beispiel des Beines deutlich, obwohl das beschriebenen Verfahren auch an allen anderen Gliedern verwendet werden kann, bei denen mittlere und äußere Gelenke vorhanden sind, beispielsweise am Arm.

Beim Bein wird das mittlere Gelenk durch das Kniegelenk gebildet, die beiden äußeren Gelenke durch das Hüftgelenk und durch das Fußgelenk. Das Hüftgelenk ist ein Kugelgelenk, so daß durch Bewegung des Oberschenkels gegenüber dem Hüftknochen der Mittelpunkt dieses Kugelgelenks bestimmt werden kann, also ein Gelenkpunkt größter Invarianz, das heißt ein bei der Bewegung der beiden Knochen gegeneinander unbeweglicher Gelenkpunkt.

In ähnlicher Weise läßt sich auch beim Fußgelenk ein solcher Punkt größter Invarianz bestimmen. Zwar ist das Fußgelenk im wesentlichen ein Gelenk, das nur eine Verschwenkung um eine Querachse ermöglicht, in geringem Umfange ist aber auch eine Drehung um die Längsachse möglich, so daß durch die Überlagerung dieser beiden Schwenkbewegungen ein Punkt bestimmt werden kann, der bei jeder Bewegung des Fußgelenkes im wesentlichen unbewegt bleibt.

Im Bereich des Knies werden zusätzlich in ähnlicher Weise Gelenkpunkte bestimmt, wobei dafür dem Chirurgen verschiedene Methoden zur Verfügung stehen können.

Wenn das Kniegelenk intakt ist und noch normale Bewegungen ermöglicht, können auch die knienahen Gelenkpunkte durch eine Bewegung der beiden angrenzenden Knochen um dieses Gelenk bestimmt werden. Zwar beschreibt das Kniegelenk eine relativ komplizierte Abroll-und Gleitbewegung, trotzdem lassen sich bei Ausführung dieser komplizierten überlagerten Bewegung und außerdem bei einer Drehung des Unterschenkels um die senkrechte Achse Punkte bestimmen, bei denen die Bewegung beim Beugen des Knies minimal wird, ein solcher Punkt maximaler Invarianz wird als Gelenkpunkt definiert.

Gemäß einer anderen Ausführungsform der Erfindung kann man Gelenkpunkte auch dadurch bestimmen, daß man diese am mittleren Gelenk durch Palpation der Gelenkflächen festlegt. Bei der Ersetzung eines mittleren Gelenkes, also beispielsweise des Kniegelenkes, muß dieser Bereich ohnehin eröffnet werden, und dann kann der Chirurg durch Tasten bestimmte markante Punkte der Gelenkflächen festlegen, beispielsweise zwischen den beiden Kondylen. Diese werden dann als Gelenkpunkte bestimmt.

Es ist auch möglich, bei einer anderen Ausführungsform der Erfindung die Gelenkpunkte der Knochen am mittleren Gelenk aus einem Datensatz zu bestimmen, der die Kontur der Gelenkfläche am mittleren Gelenk wiedergibt. Diese Kontur der Gelenkfläche kann nach dem Eröffnen des Kniegelenks beispielsweise durch einen Taster erfaßt werden, der an der Gelenkfläche entlanggeführt wird und der in verschiedenen Stellungen längs der Gelenkfläche seiner Position entsprechende Signale an eine Datenverarbeitungsanlage liefert. Diese kann auf diese Weise die Kontur der Gelenkfläche bestimmen, und anhand dieser bestimmten Kontur kann der Chirurg dann festlegen, welcher Punkt als Gelenkpunkt des mittleren Gelenkes verwendet wird.

Als Resultat dieser Bestimmung der Gelenkpunkte in den beiden äußeren Gelenken und im mittleren Gelenk lassen sich für jeden der beiden das mittlere Gelenk bildenden Knochen charakteristische Richtungen bestimmen, indem die beiden Gelenkpunkte jedes Knochens geradlinig miteinander verbunden werden. Diese charakteristischen Richtungen werden dann zur Orientierung der Prothesenteile herangezogen, das heißt anhand dieser charakteristischen Richtung wird die Neigung der Prothesenteile bestimmt, unter der diese in den Knochen eingebaut werden sollen.

Zur Bestimmung dieser charakteristischen Richtung ist es nicht notwendig, den Knochen in seiner Gesamtkontur vorher zu bestimmen, beispielsweise durch eine Computertomographie, sondern die Gelenkpunkte werden im Idealfall ausschließlich durch die kinematische Bestimmung der Gelenkpunkte im mittleren Gelenk und in den beiden äußeren Gelenken bestimmt. Nur in dem Fall, in dem das mittlere Gelenk eine solche Bestimmung durch Beschädigung nicht mehr zuläßt, tritt an die Stelle der kinematischen Bestimmung die beschriebene Bestimmung durch Palpation oder durch Aufnahme der Kontur der Gelenkfläche. In jedem Fall kann die Bestimmung der Lage des Knochens unmittelbar vor der eigentlichen Operation stattfinden, es ist nicht notwendig, in zeitlichem Abstand vor der Operation komplizierte Untersuchungen durchzuführen.

Gemäß einer bevorzugten Ausführungsform der Erfindung kann vorgesehen sein, daß man zur Orientierung der Endoprothesenteile als Anlageflächen für diese dienende Sägeebenen bestimmt, die relativ zu der charakteristischen Richtung eine vorbestimmte Orientierung einnehmen, insbesondere können diese Sägeebenen senkrecht auf der charakteristischen Richtung stehen.

Wählt man eine derartige Orientierung der Sägeebenen, an denen die Prothesenteile angelegt werden, so erhält man einen Verlauf der Beugeachse des mittleren Gelenkes, der senkrecht auf den beiden charakteristischen Richtungen der beiden am mittleren Gelenk endenden Knochen steht, und dies führt dazu, daß bei gestrecktem mittleren Gelenk die charakteristischen Richtungen dieser beiden Knochen eine gerade Linie bilden. Dies ist ein idealer Verlauf für die mechanische Belastung des Gliedes, insbesondere eines Beines, und dies kann allein aufgrund der beschriebenen Bestimmung der charakteristischen Richtungen der beiden Knochen und durch eine entsprechende Orientierung der Prothesenteile relativ zu diesen charakteristischen Richtungen erreicht werden.

Zusätzlich zur Vorbestimmung der Neigung derartiger Sägeebenen relativ zum charakteristischen Abstand kann bei einer weiteren bevorzugten Ausführungsform auch vorgesehen sein, daß die Sägeebene in einem bestimmten Abstand von dem für den jeweiligen Knochen bestimmten Gelenkpunkt am mittleren Gelenk angeordnet ist. Damit ergibt sich eine vollständige Bestimmung der Lage und Orientierung einer solchen Sägeebene aufgrund der oben beschriebenen kinematischen Bestimmung der Gelenkpunkte. Allerdings wird dies nicht in jedem Fall praktikabel sein, da sich häufig erst bei der Operation herausstellt, wie weit ein Knochen im Gelenkbereich geschädigt ist, das heißt wie weit der Knochen gelenkseitig entfernt werden muß. In diesen Fällen ist es ausreichend, wenn die Neigung der Sägeebene relativ zu der charakteristischen Richtung bestimmt wird, der Abstand vom Gelenk wird dann durch entsprechende Wahl unterschiedlicher Prothesenteile eines Satzes ausgeglichen oder durch Unterlagen, die zwischen Prothesenteil und Knochen eingefügt werden. Hier hat der Chirurg andere Möglichkeiten, diesen Abstand gegebenenfalls auszugleichen.

Im einfachsten Fall wird die kinematische Bestimmung der Lage der Gelenkpunkte vom Chirurgen dadurch vorgenommen, daß er die Knochen des Gliedes von Hand gegeneinander bewegt. Es kann aber bei einer bevorzugten Ausführungsform auch vorgesehen sein, daß die Bewegung der Knochen zur Bestimmung der Gelenkpunkte durch eine Antriebsvorrichtung ausgeführt wird.

Bei einer besonders bevorzugten Ausführungsform der Erfindung ist vorgesehen, daß zur Bestimmung der Gelenkpunkte die beiden das mittlere Gelenk bildenden Knochen sowie die beiden sich an die äußeren Gelenke anschließenden Knochen jeweils mit Markierungselementen fest verbunden werden, deren Lage im Raum durch eine Meßeinrichtung bestimmt wird, die dieser jeweiligen Lage entsprechende Signale erzeugt und einer Datenverarbeitungsanlage zuführt. Es kann sich dabei um Markierungselement und Meßeinrichtungen handeln, wie sie an sich aus der US-A-5,249,581 bekannt sind, jedoch ist hier von Bedeutung, daß sowohl die an das mittlere Gelenk anschließenden Knochen als auch die beiden an das äußere Gelenk anschließenden Knochen mit derartigen Markierungselementen verbunden sind, daß also mindestens vier Knochen mit drei eingeschlossenen Gelenken vorhanden sind, wobei jeder Knochen ein solche Markierungselement trägt, so daß man zumindest die Gelenkpunkte der beiden äußeren Gelenke bei Bewegung der Knochen relativ zu diesen Gelenken bestimmen kann.

Insbesondere kann man als Markierungselemente und Meßeinrichtung Strahlungssender beziehungsweise mehrere Strahlungsempfänger verwenden, beispielsweise Infrarotstahlungssender oder Ultraschallstrahlungssender und entsprechende Empfänger.

Die Markierungselemente können auch passive Elemente sein, beispielsweise reflektierende Kugeln, auf die von der Meßeinrichtung ausgesandte Strahlung fällt, die von der Kugeloberfläche reflektiert und dann von der Meßeinrichtung wieder aufgenommen wird. Wesentlich ist lediglich, daß die Meßeinrichtung die Lage der Markierungselemente im Raum in geeigneter Weise bestimmen kann.

In der Datenverarbeitungsanlage bestimmt man dann aufgrund der Bewegungsdaten für jedes Gelenk als Gelenkpunkt den Punkt der größten Invarianz bei der Bewegung der beiden das Gelenk bildenden Knochen.

Die so gewonnenen Daten einer charakteristischen Richtung und gegebenenfalls einer Sägeebene kann man gemäß einer bevorzugten Ausführungsform der Erfindung dazu einsetzen, daß man eine Sägeschablone relativ zu der charakteristischen Richtung des Knochens ausrichtet. Diese Ausrichtung kann beispielsweise mittels eines Roboters vorgenommen werden, der durch die Positionsdaten der Datenverarbeitungsanlage gesteuert wird.

Es ist aber gemäß einer bevorzugten Ausführungsform der Erfindung auch möglich, daß man die Ausrichtung manuell vornimmt und dabei durch Messung der Orientierung der Sägeschablone deren Orientierung relativ zu der charakteristischen Richtung laufend bestimmt. Der Chirurg muß also zur Vorbereitung eines Sägeschnittes lediglich eine entsprechende Schablone so orientieren, daß diese mit der berechneten Orientierung der Sägefläche übereinstimmt.

Günstig ist es, wenn man zur Beobachtung der Abweichung der Orientierung der Sägeschablone von der charakteristischen Richtung Differenzsignale erzeugt, die bei zutreffender Orientierung minimal sind, und wenn man diese Differenzsignale optisch oder akustisch anzeigt. Dies ermöglicht dem Chirurgen, vor der eigentlichen Operation zur Vorbereitung eines Operationsschrittes eine Sägeschablone durch Beobachten dieser Differenzsignale so einzurichten, daß ihre Orientierung mit der für die Sägefläche berechneten Orientierung übereinstimmt.

Beispielsweise kann man die Differenzsignale durch gegeneinander geneigte Linien anzeigen, die bei zutreffender Orientierung parallel zueinander verlaufen. Dabei ist es günstig, wenn die Linien sich schneiden.

Bei einer anderen Ausführungsform kann vorgesehen sein, daß man die Differenzsignale durch den Abstand von zwei parallelen Linien anzeigt, deren Abstand bei zutreffender Orientierung verschwindet.

Bei einer anderen Ausführungsform kann man die Differenzsignale durch Töne mit variierender Lautstärke oder variierender Frequenz darstellen, so daß der Chirurg allein aufgrund der Lautstärkeänderung oder der Frequenzänderung die optimale Orientierung vornehmen kann.

Dabei ist es günstig, wenn man zwei getrennte Differenzsignale für Winkelabweichungen in senkrecht zueinander stehenden Ebenen erzeugt, so daß es dem Chirurgen ohne weiteres möglich ist, die Schablone um senkrecht zueinander stehende Winkel zu verschwenken, bis die optimale Position gefunden ist.

Grundsätzlich ist es natürlich auch möglich, die so gewonnenen Daten der charakteristischen Richtung unmittelbar zu verwenden, um einen Bearbeitungsroboter zu steuern, also beispielsweise einen Sägeroboter.

Die oben angegebene Aufgabe wird mit einer Vorrichtung gemäß Anspruch 25 gelöst.

Diese Datenverarbeitungsanlage ist erfindungsgemäß so ausgebildet, daß sie aus den Signalen bei der Bewegung der Knochen um die beiden äußeren Gelenke die Punkte größter Invarianz als Gelenkpunkte bestimmt.

Vorzugsweise ist weiterhin vorgesehen, daß die Datenverarbeitungsanlage aus den Signalen bei der Bewegung der Knochen um das mittlere Gelenk zusätzlich den Punkt größter Invarianz als Gelenkpunkt des mittleren Gelenkes bestimmt.

Gemäß einer bevorzugten Ausführungsform ist vorgesehen, daß der Datenverarbeitungsanlage ein Tastinstrument zugeordnet ist, das seiner Positionierung entsprechende Signale an die Datenverarbeitungsanlage liefert. Dieses Tastinstrument kann beispielsweise verwendet werden, um einen bestimmten Punkt an der Gelenkfläche des eröffneten Gelenkes zu ertasten und dessen Lage im Raum an die Datenverarbeitungsanlage weiterzugeben. Mit diesem Tastinstrument lassen sich weiterhin eine größere Anzahl von Punkten auf der Gelenkfläche bestimmen, so daß der gesamte Verlauf einer abgetasteten Gelenkfläche an die Datenverarbeitungsanlage weitergegeben werden kann, die daraus einen Datensatz bestimmen kann, aus dem sich der gesamte Verlauf der Gelenkfläche ergibt. Schließlich läßt sich das Tastinstrument auch einsetzen, um an verwendeten Orientierungsgeräten, beispielsweise Sägeschablonen, den Verlauf der Anlagefläche für ein Sägeblatt zu bestimmen.

Die Datenverarbeitungsanlage ist so ausgebildet, daß sie aus der Lage der zwei Gelenkpunkte der beiden an das mittlere Gelenk anschließenden Knochen je eine charakteristische Richtung für den Knochen bestimmt.

Dabei ist es vorteilhaft, wenn die Datenverarbeitungsanlage zur Orientierung der Endoprothesenteile als Anlageflächen für diese dienende Sägeebenen bestimmt, die relativ zur der charakteristischen Richtung eine vorbestimmte Orientierung einnehmen, insbesondere senkrecht auf dieser charakteristischen Richtung stehen.

Bei einer weiteren bevorzugten Ausführungsform der Erfindung ist vorgesehen, daß sie eine Antriebsvorrichtung zur Bewegung der Knochen relativ zu den äußeren Gelenken und gegebenenfalls zur Bewegung relativ zum mittleren Gelenk umfaßt. Dadurch erfolgt die Bewegung der Knochen um die jeweiligen Gelenke maschinell und ermöglicht eine vollautomatische kinematische Bestimmung der Gelenkpunkte.

Die Markierungselemente und die Meßeinrichtung können als Strahlungssender beziehungsweise Strahlungsempfänger ausgebildet sein.

Der Vorrichtung kann weiterhin ein Roboter zugeordnet sein, der eine Werkzeugschablone oder ein Werkzeug relativ zu der charakteristischen Richtung ausrichtet.

Es kann weiterhin vorgesehen sein, daß einem Werkzeug oder einer Werkzeugschablone ein Markierungselement zugeordnet ist, dessen Orientierung von der Meßeinrichtung bestimmt wird, so daß dieser Orientierung entsprechende Signale an die Datenverarbeitungsanlage übertragen werden. Die Datenverarbeitungsanlage erhält somit sowohl die Positionssignale der Knochen als auch die Positionssignale des Werkzeugs oder der Werkzeugschablone, so daß die Relativpositionierung überwacht und gegebenenfalls gesteuert werden kann.

Weitere bevorzugte Ausführungsformen der erfindungsgemäßen Vorrichtung ergeben sich aus den Unteransprüchen.

Die nachfolgende Beschreibung bevorzugter Ausführungsformen der Erfindung dient im Zusammenhang mit der Zeichnung der näheren Erläuterung. Es zeigen:
- Figur 1:: eine schematische Ansicht einer Vorrichtung zur Bestimmung der charakteristischen Richtung eines Oberschenkelknochens und eines Unterschenkelknochens;
- Figur 2:: ein in einen Knochen eingesetztes Markierungselement;
- Figur 3:: eine schematische Ansicht der durch Gelenkpunkte CA, CP und CB definierten charakteristischen Richtungen eines Oberschenkels und eines Unterschenkels mit jeweiligen Sägeflächen;
- Figur 4:: eine schematische Ansicht eines mit einem Markierungselement versehenen Knochens mit einer ebenfalls mit einem Markierungselement versehenen Sägeschablone;
- Figur 5:: eine bildlich Darstellung einer Orientierungshilfe für eine Werkzeugschablone und
- Figur 6:: ein anderes Ausführungsbeispiel einer bildlichen Orientierungshilfe für eine Werkzeugschablone.

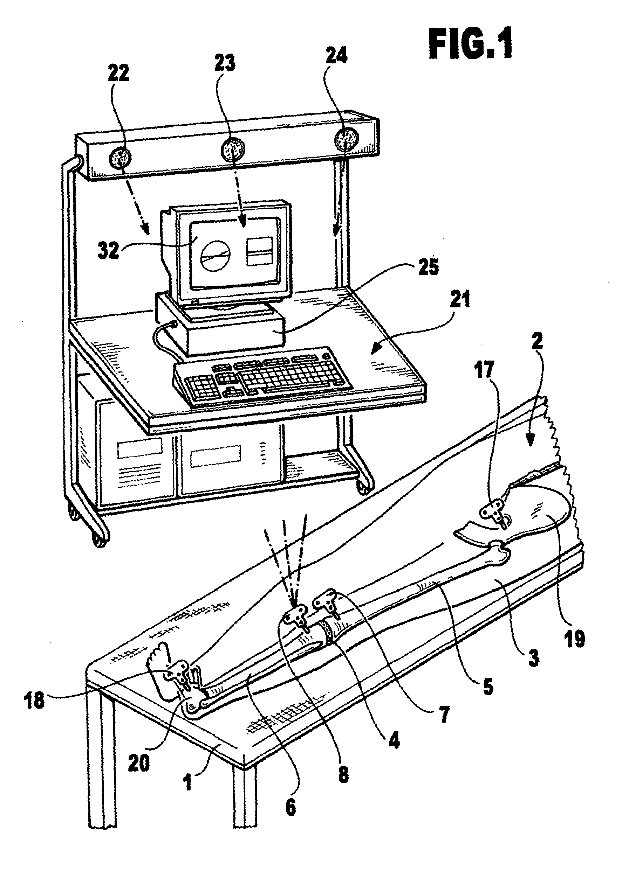

In Figur 1 ist ein auf einem Operationstisch 1 liegender Patient 2 schematisch dargestellt, bei dem in einem Bein 3 das Kniegelenk 4 durch eine Endoprothese ersetzt werden soll.

Zur Vorbereitung dieser Operation ist es notwendig, die Orientierung der zu verwendenden Prothesenteile relativ zu den Knochen zu bestimmen, also relativ zum Oberschenkelknochen 5 und zum Unterschenkelknochen 6.

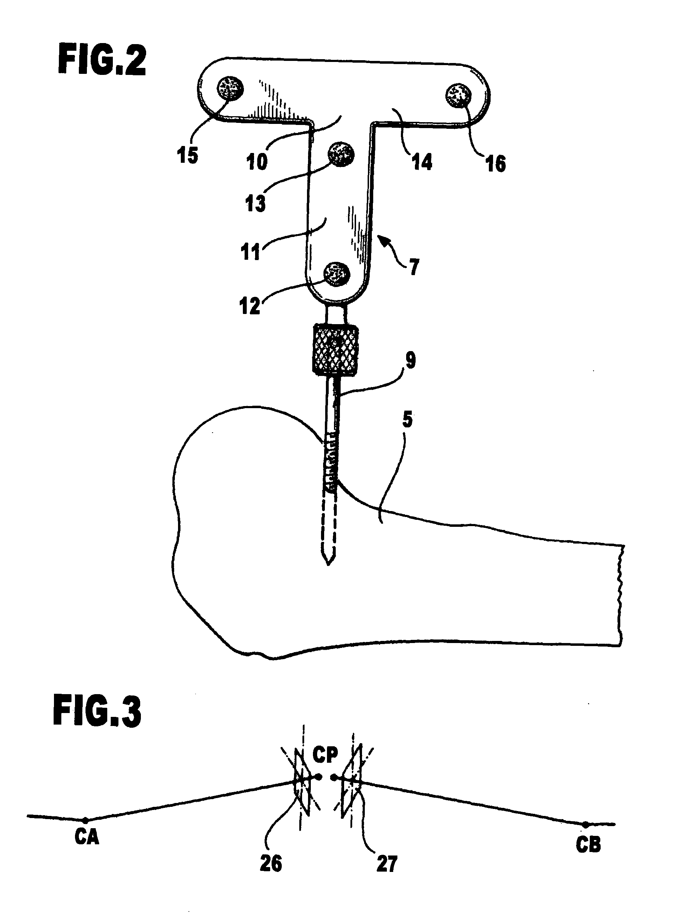

Zu diesem Zweck werden sowohl in den Oberschenkelknochen 5 als auch in den Unterschenkelknochen 6 durch kleine Einschnitte im umgebenden Gewebe hindurch Markierungselemente 7 beziehungsweise 8 eingesetzt, wie sie in Figur 2 dargestellt sind. Diese Markierungselemente 7, 8 umfassen einen in den Knochen einschraubbaren Fuß 9 in Form einer Knochenschraube und einen T-förmigen Aufsetzkörper 10, der an seinem parallel zum Fuß 9 verlaufenden Steg 11 im Abstand zueinander zwei Strahlungssender 12, 13 und an seinem an den Steg 11 anschließenden Quersteg 14 ebenfalls zwei Strahlungssender 15, 16 trägt. Diese Strahlungssender können beispielsweise Ultrarotdioden sein oder Ultraschallsender. Der Aufsetzkörper 10 kann lösbar auf den Fuß 9 aufgesetzt sein, der allerdings nur in einer ganz bestimmten Position relativ zum Fuß 9 aufgesetzt werden kann, so daß auch nach der Abnahme und nach dem Wiederaufsetzen eines solchen Aufsetzkörpers 10 die Strahlungssender 12, 13, 15, 16 relativ zum Knochen exakt dieselbe Position einnehmen wie vor dem Abnehmen.

Derartige Markierungselemente 17 und 18 werden nicht nur am Oberschenkel 5 und am Unterschenkel 6 festgelegt, sondern auch am Hüftknochen 19 und am Fußknochen 20.

An einer Konsole 21 sind im Abstand zueinander drei Empfangseinrichtungen 22, 23, 24 angeordnet, die die Strahlung empfangen, die von den Strahlungssendern 12, 13, 15, 16 ausgesandt werden. Beim Empfang von Strahlung erzeugen die Empfangseinrichtungen elektrische Signale, die einer Datenverarbeitungsanlage 25 zugeführt werden. Aufgrund der unterschiedlichen Orientierung von Markierungselementen und Empfangseinrichtungen ergeben sich Laufzeitunterschiede zwischen Aussenden und Empfangen der Strahlung, und aufgrund dieser Laufzeitunterschiede kann die Datenverarbeitungsanlage 25 bei jedem Markierungselement 7, 8, 17, 18 dessen Lage im Raum vollständig bestimmen und diese Lagedaten speichern. Es ist dadurch möglich, in der Datenverarbeitungsanlage Datensätze zu erzeugen, die der Lage der Markierungselemente und damit der fest mit ihnen verbundenen Knochen zu bestimmten Zeiten entsprechen.

Die Empfangseinrichtungen 22, 23, 24 können in unterschiedlicher Weise ausgebildet sein, sie können, wie beschrieben, die Orientierung der Markierungselemente durch Laufzeitunterschiede feststellen, grundsätzlich möglich wäre auch die Bestimmung der Orientierung durch geometrische Messung der Strahlrichtung von Strahlung, die von den Strahlungssendern 12, 13, 15, 16 ausgesandt wird. Bei anderen Ausgestaltungen können auch Markierungselemente verwendet werden, die keine Strahlungssender aufweisen, sondern Reflexionsflächen, an denen von der Empfangseinrichtung ausgesandte Strahlung reflektiert wird. Diese Reflexionsflächen können beispielsweise Kugelform haben.

Wesentlich ist lediglich, daß es aufgrund der Verwendung von mehreren Empfangseinrichtungen und mehreren Sendern oder Reflexionsflächen an den Markierungselementen möglich ist, die Lage jedes Markierungselementes im Raum eindeutig zu bestimmen.

Wenn zwei Knochen gegeneinander bewegt werden, so kann diese Bewegung somit von der Datenverarbeitungsanlage 25 in entsprechende Datensätze umgesetzt werden, die die Bahnen der Markierungselemente und dabei der Knochen bei der Bewegung bestimmen. Die Datenverarbeitungsanlage kann aus diesen Bahnen Punkte bestimmen, die bei einer solchen Bewegung von zwei Knochen relativ zu einem Gelenk unbewegt bleiben oder sich nur minimal bewegen, diese Punkte werden als Punkte maximaler Invarianz bezeichnet und als Gelenkpunkte der entsprechenden Gelenke definiert.

Im Fall des Hüftgelenkes ergibt sich ein solcher Gelenkpunkt automatisch als Mittelpunkt des als Kugelgelenk ausgebildeten Hüftgelenks, im Fall des Fußgelenkes ergibt sich ein solcher Gelenkpunkt als Schnittpunkt der Schwenkachsen des Fußgelenkes um eine quer zum Bein verlaufende Achse und um eine längs zum Bein verlaufende Achse, im Falle des Kniegelenkes ist die Situation komplizierter, da das Kniegelenk weder ein Kugelgelenk noch ein Scharniergelenk ist. Es ergeben sich aber beim Beugen des Knies und beim Drehen des Unterschenkels um dessen Längsachse Kurven, auf denen die Punkte maximaler Invarianz liegen, also im wesentlichen Kurven maximaler Invarianz, und diese nähern sich sehr stark an. Der Punkt maximaler Annäherung dieser Kurven läßt sich als Gelenkpunkt definieren, der sich bei der beschriebenen Bewegung des Oberschenkelknochens gegenüber dem Unterschenkelknochen finden läßt. Auch eine solche Berechnung wird durch die Datenverarbeitungsanlage 25 durchgeführt, so daß auf diese Weise die Datenverarbeitungsanlage sowohl im Bereich des Fußgelenkes als auch im Bereich des Hüftgelenkes als schließlich auch im Bereich des Kniegelenkes derartige Gelenkpunkte bestimmen kann.

Weiterhin berechnet die Datenverarbeitungsanlage 25 eine charakteristische Richtung für den Unterschenkel, die sich aus einer geradlinigen Verbindung des Gelenkpunktes im Knie und des Gelenkpunktes im Fußgelenk ergibt, in gleicher Weise wird für den Oberschenkel eine charakteristische Richtung bestimmt, die sich aus der geradlinigen Verbindung des Gelenkpunktes im Knie und des Gelenkpunktes in der Hüfte ergibt. Diese charakteristischen Richtungen müssen nicht unbedingt mit dem tatsächlichen Verlauf des Knochens zusammenfallen, sondern es handelt sich um virtuelle Richtungen, die sich allein aus den kinematischen Daten ergeben.

In Figur 3 ist der Verlauf dieser charakteristischen Richtungen schematisch dargestellt. Für den Oberschenkel ergibt sich diese aus der geradlinigen Verbindung des hüftnahen Gelenkpunktes CA und des knienahen Gelenkpunktes CP, für den Unterschenkel durch die geradlinige Verbindung des fußnahen Gelenkpunktes CB und des knienahen Gelenkpunktes CP.

Anhand dieser beiden, allein durch eine Bewegung des Beines des Patienten gewonnenen charakteristischen Richtungen läßt sich nun präoperativ die Orientierung einer Sägeebene bestimmen, längs welcher der Oberschenkel beziehungsweise der Unterschenkel abgetrennt werden müssen, um an dieser Sägefläche anliegend die Prothesenteile zu implantieren.

Die Datenverarbeitungsanlage bestimmt aus den so gewonnenen charakteristischen Richtungen die Orientierung dieser Sägeebenen 26, 27, die vorzugsweise senkrecht auf den charakteristischen Richtungen stehen. Dies ist in Figur 3 schematisch angedeutet. Die Orientierung der Sägeebenen wird dabei relativ zur Orientierung der Markierungselemente 7 und 8 berechnet, die wiederum stellvertretend für die Orientierung des Oberschenkels 5 und des Unterschenkels 6 sind.

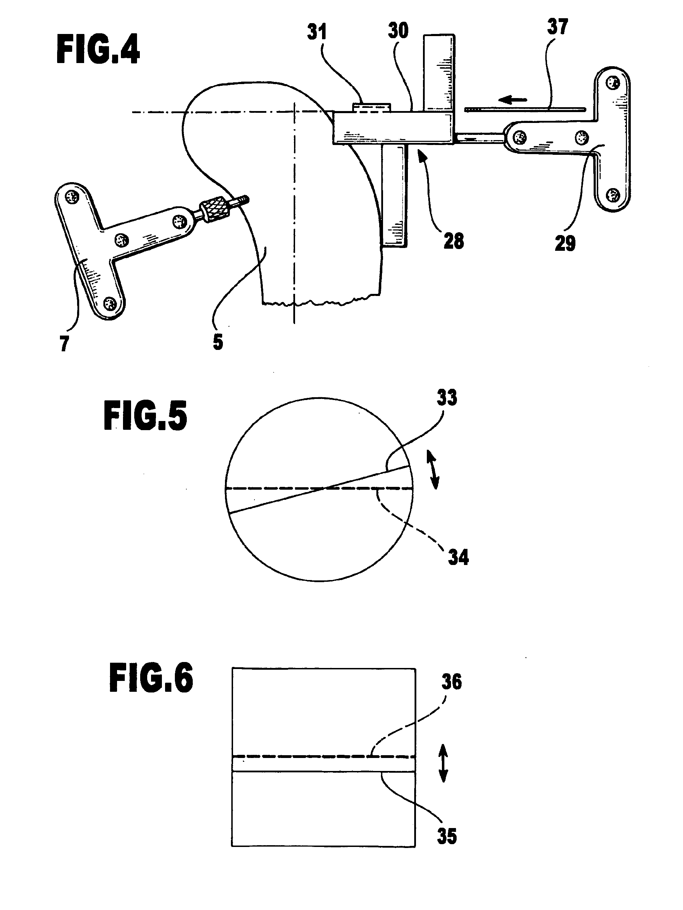

Zur Vorbereitung der Operation können die in dieser Weise gewonnenen Daten der Sägeebene nun verwendet werden, um beispielsweise eine Sägeschablone 28 relativ zu einem Knochen auszurichten. In Figur 4 wird dies anhand des Oberschenkelknochens 5 schematisch dargestellt. Der Oberschenkelknochen 5 trägt das Markierungselement 7, so daß seine Lage im Raum in der beschriebenen Weise festgestellt werden kann.

Eine Sägeschablone 28 trägt ebenfalls ein Markierungselement 29, so daß auch die Lage der Sägeschablone 28 im Raum jederzeit über die Datenverarbeitungsanlage 25 bestimmbar ist. Die Sägeschablone 28 weist eine ebene Führungsfläche 30 für ein Sägeblatt 31 auf, die Lage der Führungsfläche 30 relativ zum Markierungselement 29 läßt sich in einfacher Weise dadurch bestimmen, daß mit einem geeichten, handgeführten Tastelement die Führungsfläche 30 abgebildet wird. Dazu wird dieses Tastelement mit seiner Spitze an der Führungsfläche 30 entlanggeführt, ein mit dem Tastinstrument verbundenes Markierungselement meldet dabei alle Positionsdaten des Tastelementes zur Datenverarbeitungsanlage, die auf diese Weise die Daten der Fläche aufnehmen kann, die von der Spitze des Tastelementes abgefahren werden. Nach einer solchen Kalibrierung stehen der Datenverarbeitungsanlage die Daten zur Verfügung, um aus der Orientierung des Markierungselementes 29 die Orientierung der Führungsfläche 30 zu berechnen.

Zur richtigen Orientierung der Sägeschablone 28 muß nunmehr die Führungsfläche 30 so orientiert werden, daß sie senkrecht auf der charakteristischen Richtung des Oberschenkelknochens steht, und dies läßt sich relativ einfach dadurch bewerkstelligen, daß von der Datenverarbeitungsanlage 25 ein Differenzsignal erzeugt wird, welches der Abweichung der Orientierung der Führungsfläche 30 von der Orientierung der berechneten Sägeebene 26 entspricht. Ein solches Differenzsignal kann in verschiedener Weise für den Operateur wahrnehmbar gestaltet werden.

Zu diesem Zweck ist beispielsweise auf der Konsole 21 ein Monitor 32 angeordnet, auf dem graphische Darstellungen abgebildet werden, die ein Maß für dieses Differenzsignal sind. Ein mögliches Differenzsignal kann beispielsweise durch die Neigung von zwei geraden Linien 33, 34 gegeneinander wiedergegeben werden (Figur 5), wobei vorzugsweise der Neigungswinkel der beiden Linien dem Abweichungswinkel der Sägeebene 26 von der Führungsfläche 30 in einer Richtung entspricht. Sobald die Führungsfläche 30 so orientiert ist, daß die beiden sich schneidenden Linien 33 und 34 sich decken, ist die Führungsfläche in der entsprechenden Richtung wunschgemäß orientiert.

Bei einer anderen graphischen Darstellungsmöglichkeit wird das Differenzsignal durch den Abstand von zwei parallelen Linien 35, 36 repräsentiert (Figur 6). Wenn sich diese beiden Linien 35, 36 decken, existiert kein Differenzsignal mehr, dann sind Führungsfläche 30 und Sägeebene 26 in der entsprechenden Richtung wunschgemäß orientiert. Es ist dabei vorteilhaft, wenn die Anzeige gemäß Figur 5 und die Anzeige gemäß Figur 6 kombiniert werden, die Anzeige gemäß Figur 5 und die Anzeige gemäß Figur 6 geben dann die Neigung der Sägeebene 26 relativ zur Führungsfläche 30 in senkrecht aufeinander stehenden Richtungen an. Wenn in beiden nebeneinander angeordneten Darstellungen das Differenzsignal verschwunden ist, ist die Sägeschablone 28 wunschgemäß orientiert, diese Orientierung kann dann beispielsweise durch eingeschlagene Führungsstifte 37 fixiert werden.

Die beschriebene manuelle Orientierung der Sägeschablone 28 kann selbstverständlich bei einer anderen Ausführungsform der Erfindung auch durch einen Roboter erfolgen, der von der Datenverarbeitungsanlage 25 gemäß den dort vorhandenen Datensätzen so gesteuert wird, daß die Führungsfläche 30 parallel zur Sägeebene 26 verläuft.

Damit ist die Vorbereitung der Operation beendet, der Chirurg kann nunmehr durch Führung des Sägeblattes 31 längs der Führungsfläche 30 den Knochen mit der gewünschten Orientierung abtrennen, so daß dadurch eine Anlagefläche für ein in der Zeichnung nicht dargestelltes Prothesenteil entsteht. Dieses Prothesenteil nimmt bei Anlage an dieser Anlagefläche die gewünschte Orientierung relativ zum Knochen ein, so daß auf diese Weise eine sehr exakte Positionierung von Prothesenteilen am Knochen möglich wird.

Grundsätzlich wäre es natürlich auch möglich, daß der Sägeschnitt selbst durch den Roboter durchgeführt wird, wobei dieser dann ebenfalls durch die Datensätze gesteuert wird, die in der Datenverarbeitungsanlage 25 erzeugt werden und dort zur Verfügung stehen.

In gleicher Weise wird bei beiden an das zu ersetzende Gelenk anschließende Knochen vorgegangen, so daß beide Prothesenteile in der gewünschten Weise positioniert werden können. Es ist dadurch sichergestellt, daß nach dem Einbau der Prothesenteile die Knochen die gewünschte Orientierung einnehmen, beispielsweise in der Weise, daß die charakteristischen Richtungen beider Knochen bei gestrecktem Bein eine durchgehende gerade Linie bilden.

## Patentansprüche

1. Verfahren zur präoperativen Bestimmung der Positionsdaten von Endoprothesenteilen eines mittleren Gelenkes (4) relativ zu einem der beiden das mittlere Gelenk (4) ausbildenden Knochen (5; 6), **dadurch gekennzeichnet, daß** man die an einem äußeren Gelenk, das sich an dem dem mittleren Gelenk (4) abgewandten Ende der beiden das mittlere Gelenk (4) bildenden Knochen (5, 6) befindet, zusammenkommenden Knochen (5, 19; 6, 20) gegeneinander bewegt und **dadurch** für einen der beiden das mittlere Gelenk (4) bildenden Knochen (5; 6) einen Gelenkpunkt (CA; CB) bestimmt, daß man im Bereich des mittleren Gelenkes (4) für einen der beiden das mittlere Gelenk (4) bildenden Knochen (5; 6) einen Gelenkpunkt (CP) bestimmt, daß man aus den Positionsdaten des so gewonnenen äußeren Gelenkpunktes (CA; CB) und des so gewonnenen Gelenkpunktes (CP) im Bereich des mittleren Gelenkes (4) die Orientierung der Endoprothesenteile relativ zu einem der beiden das mittlere Gelenk (4) bildenden Knochen (5; 6) bestimmt.

2. Verfahren nach Anspruch 1, **dadurch gekennzeichnet, daß** man durch geradlinige Verbindung der beiden Gelenkpunkte (CA, CP; CB, CP) für einen der das mittlere Gelenk (4) bildenden Knochen eine für diesen charakteristische Richtung bestimmt und daß man die Orientierung der Endoprothesenteile relativ zu dieser charakteristischen Richtung bestimmt.

3. Verfahren nach einem der Ansprüche 1 oder 2, **dadurch gekennzeichnet, daß** man den Gelenkpunkt (CP) des Knochens (5; 6) am mittleren Gelenk (4) durch Bewegung der beiden das mittlere Gelenk (4) bildenden Knochen (5, 6) relativ zueinander bestimmt.

4. Verfahren nach einem der Ansprüche 1 oder 2, **dadurch gekennzeichnet, daß** man den Gelenkpunkt (CP) des Knochens (5; 6) am mittleren Gelenk (4) durch Palpation der Gelenkflächen festlegt.

5. Verfahren nach einem der Ansprüche 1 oder 2, **dadurch gekennzeichnet, daß** man den Gelenkpunkt (CP) des Knochens (5; 6) am mittleren Gelenk (4) an einem Datensatz bestimmt, der die Kontur der Gelenkfläche am mittleren Gelenk (4) wiedergibt.

6. Verfahren nach Anspruch 5, **dadurch gekennzeichnet, daß** man den Datensatz der Kontur der Gelenkfläche durch Abtasten der Gelenkfläche und Speichern einer Vielzahl von Positionsdaten von Punkten auf der Gelenkfläche bestimmt.

7. Verfahren nach einem der Ansprüche 2 bis 6, **dadurch gekennzeichnet, daß** man zur Orientierung der Endoprothesenteile eine als Anlagefläche für diese dienende Sägeebene (26; 27) bestimmt, die relativ zu der charakteristischen Richtung eine vorbestimmte Orientierung einnimmt.

8. Verfahren nach Anspruch 7, **dadurch gekennzeichnet, daß** die Sägeebene (26; 27) senkrecht auf der charakteristischen Richtung steht.

9. Verfahren nach Anspruch 7 oder Anspruch 8, **dadurch gekennzeichnet, daß** die Sägeebene (26; 27) in einem bestimmten Abstand von dem für den Knochen (5; 6) bestimmten Gelenkpunkt (CP) am mittleren Gelenk (4) angeordnet ist.

10. Verfahren nach einem der voranstehenden Ansprüche, **dadurch gekennzeichnet, daß** die Bewegung der Knochen (5, 19; 6, 20) zur Bestimmung der Gelenkpunkte durch eine Antriebsvorrichtung ausgeführt wird.

11. Verfahren nach einem der voranstehenden Ansprüche, **dadurch gekennzeichnet, daß** zur Bestimmung der Gelenkpunkte einer der das mittlere Gelenk (4) bildenden Knochen (5; 6) sowie der sich an das äußere Gelenk anschließende Knochen (19; 20) jeweils mit Markierungselementen (7, 17; 8, 18) fest verbunden werden, deren Lage im Raum durch eine Meßeinrichtung (22, 23, 24) bestimmt wird, die dieser jeweiligen Lage entsprechende Signale erzeugt und einer Datenverarbeitungsanlage (25) zuführt.

12. Verfahren nach Anspruch 11, **dadurch gekennzeichnet, daß** man als Markierungselemente (7, 17; 8, 18) und Meßeinrichtung (22, 23, 24) Strahlungssender oder reflektierende Flächen beziehungsweise mehrere Strahlungsempfänger verwendet.

13. Verfahren nach Anspruch 11 oder 12, **dadurch gekennzeichnet, daß** man in der Datenverarbeitungsanlage (25) für das Gelenk als Gelenkpunkt den Punkt der größten Invarianz bei der Bewegung der beiden das Gelenk bildenden Knochen bestimmt.

14. Verfahren nach einem der Ansprüche 2 bis 13, **dadurch gekennzeichnet, daß** man zur Markierung einer Sägeebene (26; 27) eine Sägeschablone (28) relativ zu der charakteristischen Richtung des Knochens ausrichtet.

15. Verfahren nach Anspruch 14, **dadurch gekennzeichnet, daß** man die Ausrichtung mittels eines Roboters vornimmt.

16. Verfahren nach Anspruch 14, **dadurch gekennzeichnet, daß** man die Ausrichtung manuell vornimmt und dabei durch Messung der Orientierung der Sägeschablone (28) deren Orientierung relativ zu der charakteristischen Richtung laufend bestimmt.

17. Verfahren nach Anspruch 16, **dadurch gekennzeichnet, daß** man zur Beobachtung der Abweichung der Orientierung der Sägeschablone (28) zur charakteristischen Richtung Differenzsignale erzeugt, die bei zutreffender Orientierung minimal sind, und daß man diese Differenzsignale optisch oder akustisch anzeigt.

18. Verfahren nach Anspruch 17, **dadurch gekennzeichnet, daß** man die Differenzsignale durch gegeneinander geneigte Linien anzeigt, die bei zutreffender Orientierung parallel zueinander verlaufen.

19. Verfahren nach Anspruch 18, **dadurch gekennzeichnet, daß** die Linien sich schneiden.

20. Verfahren nach Anspruch 17, 18 oder 19, **dadurch gekennzeichnet, daß** man die Differenzsignale durch den Abstand von zwei parallelen Linien anzeigt, deren Abstand bei zutreffender Orientierung verschwindet.

21. Verfahren nach Anspruch 17, **dadurch gekennzeichnet, daß** man die Differenzsignale durch Töne mit variierender Lautstärke oder Frequenz darstellt.

22. Verfahren nach einem der Ansprüche 17 bis 21, **dadurch gekennzeichnet, daß** man zwei getrennte Differenzsignale für Winkelabweichungen in senkrecht zueinander stehenden Ebenen erzeugt.

23. Verfahren nach einem der Ansprüche 2 bis 13, **dadurch gekennzeichnet, daß** man die anhand der charakteristischen Richtung bestimmten Positionsdaten zur Steuerung eines Bearbeitungsroboters verwendet.

24. Verfahren nach einem der Ansprüche 2 bis 23, **dadurch gekennzeichnet, daß** die charakteristischen Richtungen bei beiden das mittlere Gelenk (4) ausbildenden Knochen (5, 6) in gleicher Weise bestimmt werden.

25. Vorrichtung zur präoperativen Bestimmung der Positionsdaten von Endoprothesenteilen eines mittleren Gelenkes (4) relativ zu einem der das mittlere Gelenk (4) bildenden Knochen (5; 6) mit an den Knochen festlegbaren Markierungselementen (7, 17; 8, 18), einer Meßeinrichtung (22, 23, 24) zur Bestimmung der Lage der Markierungselemente (7, 17; 8, 18) im Raum und mit einer Datenverarbeitungsanlage (25), der von der Meßeinrichtung (22, 23, 24) den Positionsdaten der Markierungselemente (7, 17; 8, 18) entsprechende Signale zugeführt werden, **dadurch gekennzeichnet, daß** mindestens je ein Markierungselement (7, 17; 8, 18) für einen der beiden das mittlere Gelenk (4) bildenden Knochen (5; 6) sowie für einen der beiden an diesen anschließenden, mit diesem über ein äußeres Gelenk verbundenen Knochen (19; 20) vorgesehen ist, daß die Datenverarbeitungsanlage (25) aus den Signalen bei der Bewegung der Knochen (6, 20; 5, 19) um das äußere Gelenk den Punkt größter Invarianz als äußeren Gelenkpunkt (CA; CB) bestimmt und daß die Datenverarbeitungsanlage (25) aus der Lage des äußeren Gelenkpunktes (CA; CB) und aus der Lage eines inneren Gelenkpunkts (CP) der beiden das mittlere Gelenk (4) bildenden Knochen (5, 6) die Orientierung der Endoprothesenteile relativ zu diesem Knochen (5; 6) bestimmt.

26. Vorrichtung nach Anspruch 25, **dadurch gekennzeichnet, daß** die Datenverarbeitungsanlage (25) aus dem äußeren Gelenkpunkt (CA; CB) und aus dem inneren Gelenkpunkt (CP) durch geradlinige Verbindung dieser Punkte eine charakteristische Richtung für den entsprechenden Knochen (5; 6) bestimmt.

27. Vorrichtung nach Anspruch 25 oder 26, **dadurch gekennzeichnet, daß** die Datenverarbeitungsanlage (25) aus den Signalen bei der Bewegung der Knochen (5; 6) um das mittlere Gelenk (4) zusätzlich den Punkt größter Invarianz als Gelenkpunkt (CP) des mittleren Gelenks (4) bestimmt.

28. Vorrichtung nach einem der Ansprüche 25 bis 27, **dadurch gekennzeichnet, daß** der Datenverarbeitungsanlage (25) ein Tastinstrument zugeordnet ist, das seiner Positionierung entsprechende Signale an die Datenverarbeitungsanlage (25) liefert.

29. Vorrichtung nach Anspruch 28, **dadurch gekennzeichnet, daß** die Datenverarbeitungsanlage (25) aus einer Vielzahl derartiger Signale, die durch Anlage des Tastinstruments an einer Gelenkfläche des an das mittlere Gelenk (4) anschließenden Knochens (5; 6) erzeugt worden sind, einen den Verlauf der Gelenkfläche beschreibenden Datensatz erzeugt.

30. Vorrichtung nach einem der Ansprüche 26 bis 29, **dadurch gekennzeichnet, daß** die Datenverarbeitungsanlage (25) zur Orientierung der Endoprothesenteile eine als Anlagefläche für diese dienende Sägeebene (26; 27) bestimmt, die relativ zu der charakteristischen Richtung eine vorbestimmte Orientierung einnimmt.

31. Vorrichtung nach Anspruch 30, **dadurch gekennzeichnet, daß** die Sägeebene (26; 27) senkrecht auf der charakteristischen Richtung steht.

32. Vorrichtung nach Anspruch 30 oder 31, **dadurch gekennzeichnet, daß** die Sägeebene (26; 27) in einem bestimmten Abstand von dem für den Knochen (5; 6) bestimmten Gelenkpunkt (CP) am mittleren Gelenk (4) angeordnet ist.

33. Vorrichtung nach einem der Ansprüche 25 bis 32, **dadurch gekennzeichnet, daß** sie eine Antriebsvorrichtung zur Bewegung der Knochen relativ zu dem äußeren Gelenk und gegebenenfalls zur Bewegung relativ zum mittleren Gelenk umfaßt.

34. Vorrichtung nach einem der Ansprüche 25 bis 33, **dadurch gekennzeichnet, daß** die Markierungselemente (7, 17; 8, 18) und die Meßeinrichtung (22, 23, 24) als Strahlungssender oder reflektierende Fläche beziehungsweise Strahlungsempfänger ausgebildet sind.

35. Vorrichtung nach einem der Ansprüche 26 bis 34, **dadurch gekennzeichnet, daß** ihr ein Roboter zugeordnet ist, der eine Werkzeugschablone oder ein Werkzeug relativ zu der charakteristischen Richtung ausrichtet.

36. Vorrichtung nach einem der Ansprüche 25 bis 34, **dadurch gekennzeichnet, daß** einem Werkzeug oder einer Werkzeugschablone (28) ein Markierungselement (29) zugeordnet ist, dessen Orientierung von der Meßeinrichtung (22, 23, 24) bestimmt wird, so daß dieser Orientierung entsprechende Signale an die Datenverarbeitungsanlage (25) übertragen werden.

37. Vorrichtung nach Anspruch 36, **dadurch gekennzeichnet, daß** die Datenverarbeitungsanlage (25) die Orientierung des Werkzeuges oder der Werkzeugschablone (28) relativ zu der charakteristischen Richtung bestimmt.

38. Vorrichtung nach Anspruch 37, **dadurch gekennzeichnet, daß** die Datenverarbeitungsanlage (25) zur Beobachtung der Abweichung der Orientierung der Sägeschablone (28) zur charakteristischen Richtung Differenzsignale erzeugt, die bei zutreffender Orientierung minimal sind, und daß sie diese Differenzsignale optisch oder akustisch anzeigt.

39. Vorrichtung nach Anspruch 38, **dadurch gekennzeichnet, daß** die Differenzsignale durch gegeneinander geneigte Linien (33, 34) angezeigt werden, die bei zutreffender Orientierung parallel zueinander verlaufen.

40. Vorrichtung nach Anspruch 39, **dadurch gekennzeichnet, daß** die Linien (33, 34) sich schneiden.

41. Vorrichtung nach Anspruch 38, 39 oder 40, **dadurch gekennzeichnet, daß** man die Differenzsignale durch den Abstand von zwei parallelen Linien (35, 36) anzeigt, deren Abstand bei zutreffender Orientierung verschwindet.

42. Vorrichtung nach Anspruch 38, **dadurch gekennzeichnet, daß** die Differenzsignale durch Töne mit variierender Lautstärke oder Frequenz dargestellt werden.

43. Vorrichtung nach einem der Ansprüche 38 bis 42, **dadurch gekennzeichnet, daß** sie zwei getrennte Differenzsignale für Winkelabweichungen in senkrecht zueinander stehenden Ebenen erzeugt.

44. Vorrichtung nach einem der Ansprüche 25 bis 43, **dadurch gekennzeichnet, daß** mindestens je ein Markierungselement (7, 17; 8, 18) für die beiden das mittlere Gelenk (4) bildenden Knochen (5; 6) sowie für die beiden an diese anschließenden, mit diesen über ein äußeres Gelenk verbundenen Knochen (19; 20) vorgesehen sind.
